# EUROPÄISCHE PATENTSCHRIFT

(11) **EP 0 488 192 B1**
(45) Veröffentlichungstag und Bekanntmachung des Hinweises auf die Patenterteilung: **30.08.1995**
(21) Anmeldenummer: 91120220.8
(22) Anmeldetag: 26.11.1991
(51) Int. Cl.: A61B 6/00

(54) **Kalibrierphantom für Knochenmineralmessungen an der Lendenwirbelsäule**
Determination of vertebral bone mineral density at the lumbar region by means of a calibration phantom
Fantôme d'étalonnage destiné à la mesure du contenu de minéraux dans la partie lombaire de la colonne vertébrale

(30) Priorität: 26.11.1990 DE 9016046 U
(43) Veröffentlichungstag der Anmeldung: 03.06.1992
(73) Patentinhaber: Kalender, Willi, Dr., D-91096 Möhrendorf (DE)
(72) Erfinder: Kalender, Willi, Dr., D-91096 Möhrendorf (DE)

(56) Entgegenhaltungen:
- EP-A- 0 218 367
- EP-A- 0 409 698
- US-A- 4 873 707

## Beschreibung

Die Osteoporose, eine schwerwiegende Alterserkrankung des Skeletts, gewinnt durch die stetig steigende Lebenserwartung eine immer größere Bedeutung. Die jährlichen Kosten im Gesundheitswesen aufgrund der Osteoporose werden weltweit mit mehreren Milliarden veranschlagt, in der Bundesrepublik rechnet man mit ca. 1 Milliarde Kosten. Entsprechend sind die klinischen und meßtechnischen Anstrengungen bezüglich einor Frühdiagnose und effizienten Therapiekontrolle sehr groß.
Zwei Verfahren haben sich zur Messung des Knochenmineralgehaltes als besonders geeignet erwiesen:
Die Photonenabsorptiometrie (DXA, Dual Energy X-ray Absorptiometry) und die quantitative Computertomographie (QCT). Für beide Meßverfahren sind weltweit jeweils mehr als 1000 Anlagen von unterschiedlichen Herstellern in Betrieb.

Ein besonderes Problem und erhebliche Kontroversen haben sich daraus ergeben, daß die Ergebnisse, die an unterschiedlichen Geräten gewonnen wurden, nicht übereinstimmen, bzw, schwer vergleichbar sind. Insbesondere bei den DXA-Geräten werden voll jedem Hersteller andere Phantome eingesetzt, die sowohl zur Kalibrierung als auch zur Qualitätskontrolle benutzt worden. Dadurch daß verschiedene Materialien und unterschiedliche Geometrien zum Einsatz kommen, ist die Kalibrierung unterschiedlich. Es ergeben sich Differenzen bei Messungen gleicher Objekte oder Patienten auf unterschiedlichen Apparaten. Die angebotenen Phantome bieten meistens homogenen Knochen ohne Unterscheidung von spongiösen und kortikalen Anteilen an. Durch die meist anthropomorphe Formgebung ist eine Überprüfung, ob die gemessenen Flächenwerte und die damit verknüpften Flächendichtewerte korrekt sind, nicht möglich.

Bei der QCT werden von den Herstellern keine körperähnlichen Phantome bereitgestellt. Es gibt lediglich einige kleinere Firmen, die solche Phantome anbieten. Diese sind aber nicht für den direkten Vergleich mit der DXA geeignet; auch bieten sie keine Kortikalisstrukturen an.

Da bisher nur herstellerspezifische Lösungen für jeweils nur einen Gerätetyp angeboten wurden, resultierten Kontroversen, aber bisher keine akzeptierte Lösung des Problems 'Quervergleich unterschiedlicher Geräte. Zusätzlich ist anzumerken, daß die von kleinen Spezialfirmen bereitgestellten Phantome sehr teuer sind, zum Teil wegen ihrer Komplexität unhandlich sind und nicht alle gewünschten Testmöglichkeiten bieten.

Aus EP-A-0 218 367 ist ein Phantom zur Kalibrierung und Kontrolle eines Computertomographen zur Bestimmung des Knochenmineralgehaltes bekannt, das in einem Gehäuse einen zylindrischen Körper aufweist.

Der Erfindung liegt die Aufgabe zugrunde, ein Phantom zur Kalibrierung und Kontrolle von Geräten zur Bestimmung des knochenmineralgehaltes zu schaffen, das in beiden Gerätetypen eingesetzt werden kann. Die Benutzung eines einheitlichen Phantoms mit klar definierter, angenähert anthropomorpher Geometrie und einheitlichen Materialien soll zu einer Standardisierung führen und zum Teil unnötige Kontroversen eliminieren.

Diese Aufgabe ist erfindungsgemäß dadurch gelöst, daß ein einen Wirbel nachbildender Grundkörper, der exakt geometrisch beschreibbar und gleichzeitig anthropomorph ist und bezüglich der Schwächung von Röntgenstrahlung aus gewebe- bzw. wasseräquivalentem Materialien besteht, entworfen wurde.
Das Phantom besteht dem nach aus gewebe-, bzw. wasseräquivalenten Materialien. Hierzu werden Epoxydharze als Basismaterial eingesetzt, denen unterschiedliche Chemikalien in definierter Konzentration beigemischt werden, um Feststoffe Zu erhalten, die in ihren Eigenschaften bezüglich der Schwächung von Röntgenstrahlen Wasser, bzw. Weich- und Knochengeweben äquivalent sind. Der Phantomgrundkörper hat in einer typischen Ausführung einen ovalen Querschnitt von 18 cm x 28 cm und eine Tiefe von ca. 12 cm. Zusätze (z.B. Ringe oder Platten) aus wasser- oder fettäquivalentem Material können hinzugefügt werden. Die Seiten können leicht abgeflacht werden, um eine stabile seitliche Lagerung zu ermöglichen. Das Phantom ist damit von Größe und Masse (< 5 kg) handlich. Es kann im wesentlichen aus drei Abschnitten bestehen, die jeweils einen Wirbelkörper mit unterschiedlichen Dimensionen und Mineraldichten enthalten.

Durch die exakte geometrische Definition der einzelnen Strukturen, wobei eine annähernd anthropomorphe Gestalt beibehalten wurde, sind alle Größen berechenbar und damit bei korrekter Herstellung auch die wahren Werte der einzelnen Meßgrößen angebbar.

Mit der DXA sind sowohl a.p. (anterior-posterior) und laterale Messungen möglich. Es können dabei die Fläche (cm²), die Flächendichte (g/cm²), die Masse (g) und Linearität der Messung überprüft werden.

In der QCT können sowohl spongiöse als auch kortikale Dichte gemessen und die Linearität überprüft werden. Zusätzlich ist über eine Bohrung in der mittleren Scheibe die Möglichkeit gegeben, die Positioniergenauigkeit des Gerätes zu überprüfen.

Die Erfindung ist nachfolgend an Hand eines Ausführungsbeispiels näher erläutert. Es zeigen:
- Fig. 1:: eine laterale Projektion der 'Knochenstrukturen' des Phantoms nach der Erfindung aus drei Abschnitten, und

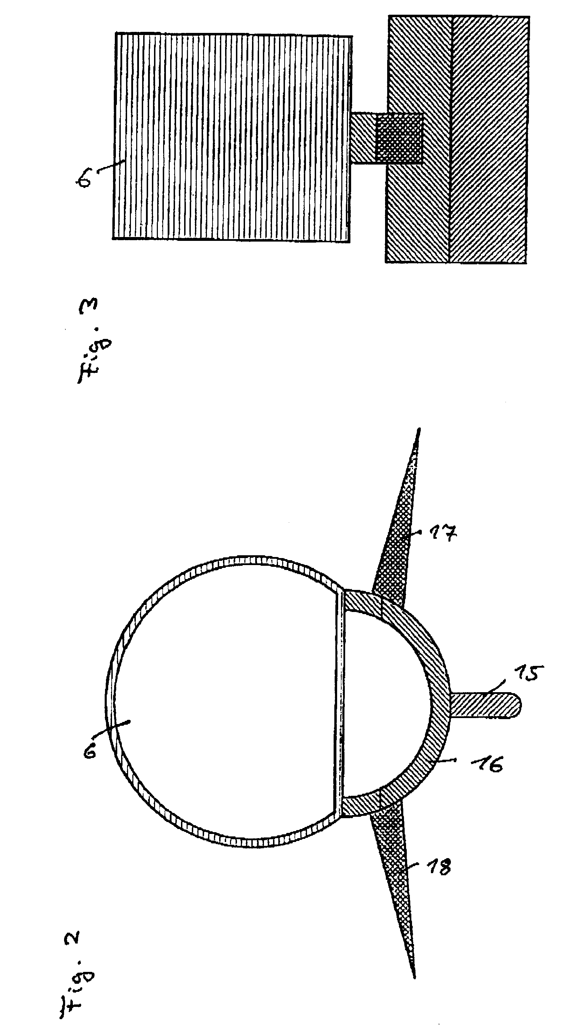

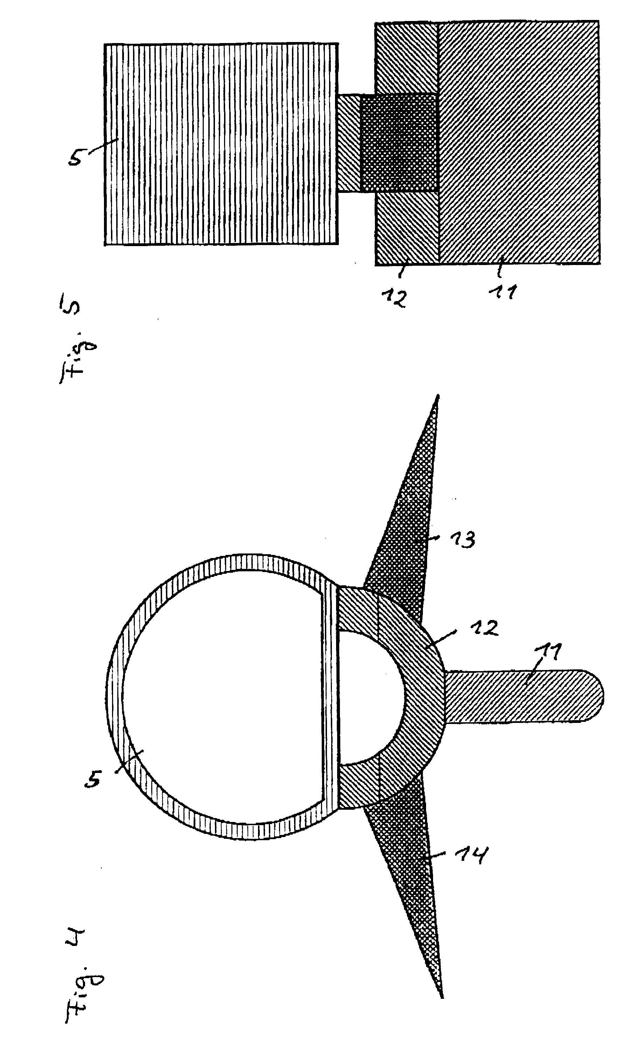

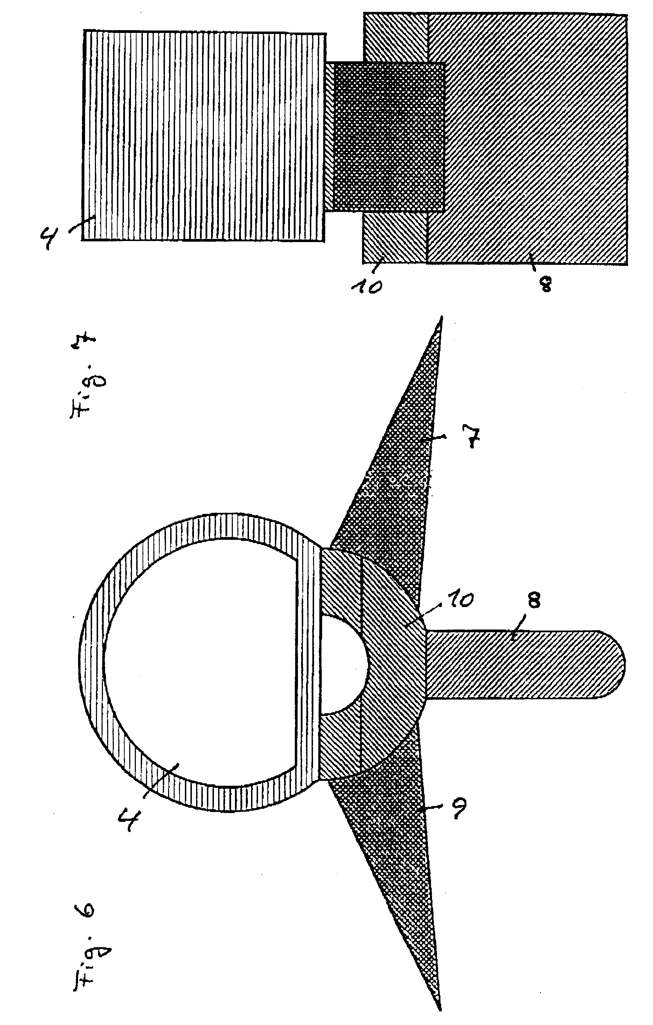

- Fig. 2-7:: jeweils zwei Ansichten der Abschnitte gemäß Fig. 1.

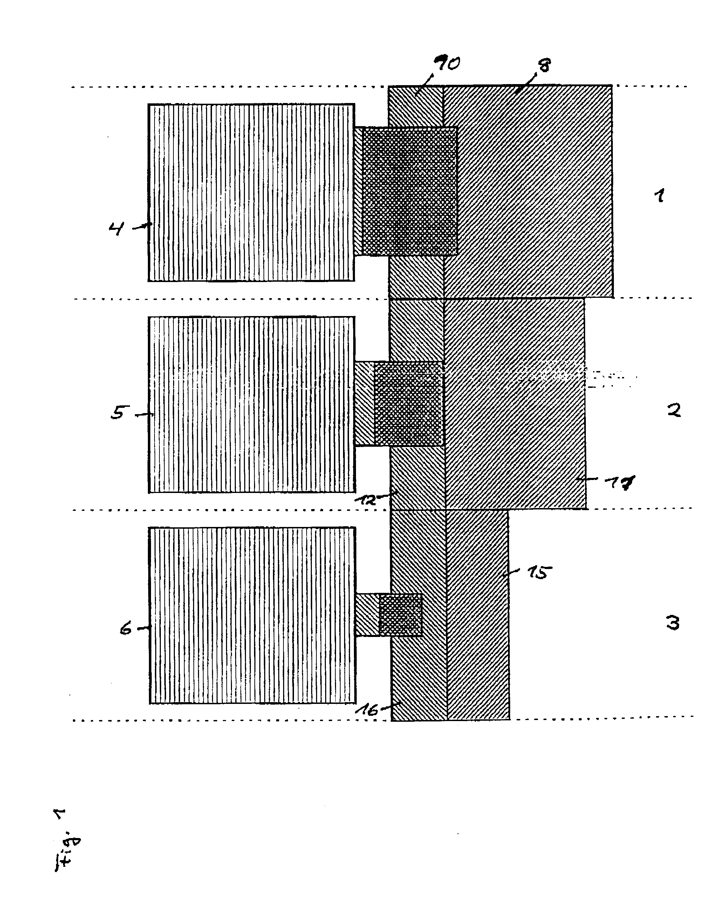

Das Phantom gemäß Fig. 1 besteht aus drei Abschnitten 1 bis 3, von denen jeder einen Grundkörper 4,5,6 aus gewebe- bzw. wasseräquivalentem Material aufweist, der einen Wirbel nachbildet. An die Grundkörper 4,5,6 sind Zusatzkörper 7 bis 18 aus gewebe- bzw. wasseräquivalentem Material zur besseren Nachbildung den Wirbeln angebaut.

Die Komponenten 4,7-10; 5,11-14; 6,15-18 weisen jeweils unterschiedliche Dimensionen und Dichten zur Nachbildung unterschiedlich dimensionierter Wirbel auf.

Eine Sonderform des Phantoms, das für den ausschließlichen Einsatz in der QCT vorgesehen ist, besteht nur aus dem mittleren Phantomabschnitt, z.B. mit einer Tiefe von 3 cm. In dieser Version ist die Spongiosa herausnehmbar; es stehen Einsätze mit unterschiedlichen Mineraldichten und Fettanteilen zur Verfügung.

## Patentansprüche

1. Phantom zur Kontrolle von Geräten zur Bestimmung des Knochenmineralgehaltes mit einem einen Wirbelkörper nachbildenden Grundkörper (4, 5, 6), der exakt geometrisch beschreibbar und gleichzeitig anthropomorph ist und bezüglich der Schwächung von Röntgenstrahlung aus gewebe- bzw. wasseräquivalentem Materialien besteht.

2. Phantom nach Anspruch 1, bei dem an den Grundkörper anbaubare Zusatzkörper (7-18) vorhanden sind.

3. Phantom nach Anspruch 1. bzw. 2., das aus mehreren Abschnitten besteht, die Grundkörper mit unterschiedlichen Dimensionen und Dichten enthalten.

4. Phantom nach Anspruch 3 mit drei Abschnitten.

## Claims

1. Phantom to control bone densitometry apparati containing a basic structure which resembles a vertebra and which offers the following characteristics: it is describable geometrically in exact form, it is anthropomorphic, and it consists of materials which are tissue or water equivalent with respect to the attenuation of x-rays.

2. Phantom according to claim 1. with structures added to the basic structure.

3. Phantom according to claim 1. and 2., respectively, built up in several sections containing basic and added structures of different dimensions and densities.

4. Phantom according to claim 3 built up in three sections

## Revendications

1. Il s'agit d'un phantome pour contrôler des appareils quantifiant la contenance minérale des os avec un corps de base représentant une vertèbre qui est décrit géométriquement exactement et en même temps anthropomorphe et, en ce qui concerne l'absorption des rayons X, fait des mâteriaux équivalents a l'eau ou aux tissues.

2. Phantom mêmes performances à dessus (voir 1) corps de bases avec accessoires en plus.

3. Phantom mêmes performances à dessus (voir 1 et 2) corps de base identique (voir 1 et 2) constitué de strates, de différentes dimensions et de différentes densités.

4. Phantom mêmes performances à dessus (voir 3) constitué de trois strates.
